# EUROPEAN PATENT SPECIFICATION

(11) **EP 3 463 186 B1**
(45) Date of publication and mention of the grant of the patent: **19.08.2020**
(21) Application number: 17729558.1
(22) Date of filing: 29.05.2017
(51) Int. Cl.: A61F 2/16

(54) **ACCOMMODATIVE INTRAOCULAR LENS**
AKKOMMODATIONSFÄHIGE INTRAOKULARLINSE
LENTILLE INTRAOCULAIRE ACCOMMODATIVE

(30) Priority: 01.06.2016 US 201615170417; 29.12.2016 US 201662439992 P
(43) Date of publication of application: 10.04.2019
(73) Proprietor: Rainbow Medical Ltd., 4614002 Herzliya (IL)
(72) Inventor: SOHN, Zev, 44853 Ginot Shomron (IL); GROSS, Yossi, 73160 Moshav Mazor (IL)
(74) Representative: White, Duncan Rohan
(86) International application number: PCT/IL2017/050594
(87) International publication number: WO 2017/208230

(56) References cited:
- US-A- 6 013 101
- US-A1- 2014 309 735

## Description

### FIELD OF THE APPLICATION

The present invention relates generally to implantable medical devices, and specifically to intraocular lenses.

### BACKGROUND OF THE APPLICATION

Accommodating intraocular lenses (AIOLs) allow the eye to focus at different distances. The Crystalens® (Bausch & Lomb, Rochester, NY, USA) is an AIOL that has received FDA approval in the United States.

US Patent Application Publication 2011/0071628 to Gross et al. describes an accommodating intraocular lens (AIOL) implant that includes at least an anterior floating lens complex and a posterior lens complex, each of which comprises one or more optical elements, and a frame comprising one or more levers, which are coupled to the frame and the anterior floating lens complex. The levers are configured to leverage motion of the frame to move the anterior floating lens complex with respect to the posterior lens complex. Other embodiments are also described.

PCT Publication WO 2015/198236 to Sohn et al., describes an accommodating intraocular lens implant that includes an anterior floating lens unit, a posterior lens unit, an anterior rim complex disposed such that the anterior floating lens unit is movable toward and away from the anterior rim complex. A plurality of levers are in jointed connection with: the anterior floating lens unit at respective first longitudinal sites along the levers, the anterior rim complex at respective second longitudinal sites along the levers, and the posterior lens unit at respective third longitudinal sites along the levers. For each of the levers, (a) a line defined by the second and third longitudinal sites, if projected onto a plane defined by a radially-outer perimeter of the lens implant, and (b) a line tangential to the radially-outer perimeter of the lens implant at a circumferential site of the perimeter circumferentially corresponding to the third longitudinal site, form an angle of between 75 and 105 degrees. US 2014/309735 A1 describes another AIOL.

### SUMMARY OF THE APPLICATION

The invention is defined by the appended claims.

### BRIEF DESCRIPTION OF THE DRAWINGS

Figs. 1A-C are schematic illustrations of an accommodative intraocular lens implant, in accordance with an application of the present invention;
Figs. 2A-C are schematic illustrations of the accommodative intraocular lens implant of Figs. 1A-C implanted in a natural capsular bag of the eye, in accordance with an application of the present invention;
Figs. 3A-B are schematic illustration of components of the lens implant of Figs. 1A-C and 2A-C prior to assembly and after assembly, respectively, in accordance with an application of the present invention;
Figs. 4A-B are schematic cross-sectional illustrations of the lens implant of Figs. 1A-C and 2A-C in a fully-unaccommodated state and a fully-accommodated state, respectively, in accordance with an application of the present invention;
Fig. 5A-C are schematic illustrations of one lever and one anterior rim link of the lens implant of Figs. 1A-C and 2A-C, in accordance with an application of the present invention;
Fig. 6 is a schematic illustration of another configuration of one anterior rim link of the lens implant of Figs. 1A-C and 2A-C, in accordance with an application of the present invention;
Fig. 7 is a schematic anterior view of first and second anterior components of the lens implant of Figs. 1A-C and 2A-C, in accordance with an application of the present invention;
Fig. 8 is a schematic anterior view of the lens implant of Figs. 1A-C and 2A-C, in accordance with an application of the present invention;
Fig. 9 is a schematic illustration of the removable disposal of a second posterior component of the lens implant of Figs. 1A-C and 2A-C in a first introducer tube, in accordance with an application of the present invention;
Fig. 10 is a schematic illustration of the removable disposal of a posterior lens rim of a first posterior component of the lens implant of Figs. 1A-C and 2A-C in a second introducer tube, in accordance with an application of the present invention;
Fig. 11 is a schematic illustration of the removable disposal of an anterior assembly of the lens implant of Figs. 1A-C and 2A-C in a third introducer tube, in accordance with an application of the present invention; and
Figs. 12A-M are schematic illustrations of a method of implanting the lens implant of Figs. 1A-C and 2A-C, in accordance with an application of the present invention.

### DETAILED DESCRIPTION OF APPLICATIONS

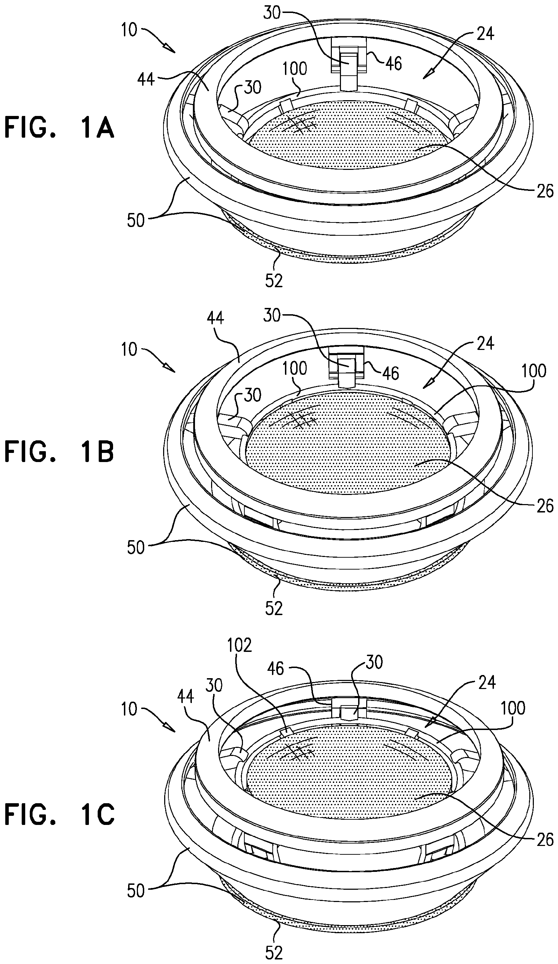

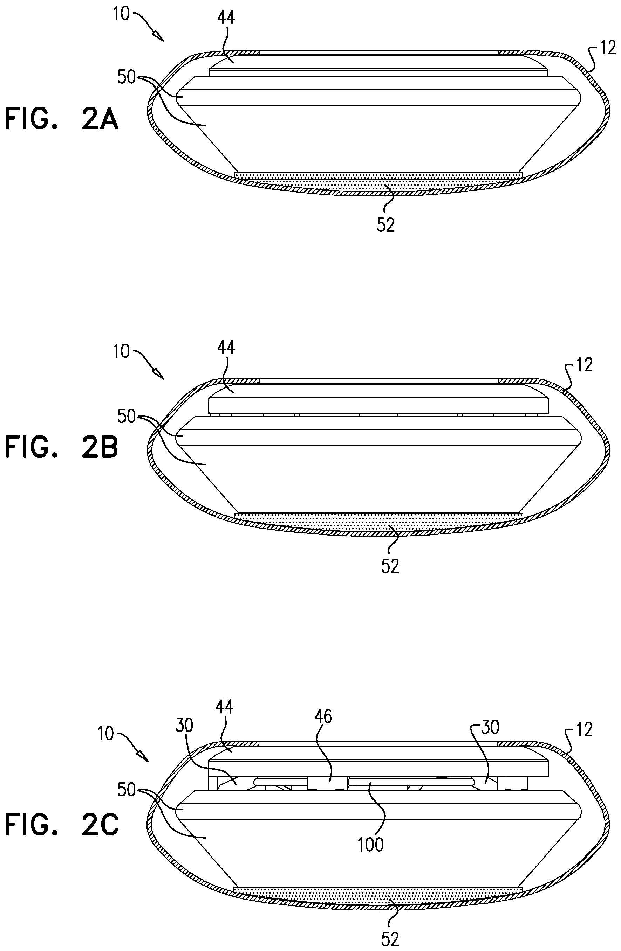

Figs. 1A-C and 2A-C are schematic illustrations of an accommodative intraocular lens implant 10, in accordance with an application of the present invention. Figs. 1A-C are isometric views of the lens implant. Figs. 2A-C are side views showing the lens implant implanted in a natural capsular bag 12 of the eye. Figs. 1A and 2A show lens implant 10 in a fully-unaccommodated state, Figs. 1B and 2B show lens implant 10 in a partially-accommodated state, and Figs. 1C and 2C show the lens implant in a fully-accommodated state. Although only these three states are shown in these and some of the other figures, lens implant 10 is configured to assume a continuous range of accommodation between the fully-unaccommodated state and the fully-accommodated state. The fully-accommodated state provides near vision, the fully-unaccommodated state provides distance vision, and partially-accommodated states therebetween provide intermediate vision. The lens implant is configured to reach the fully-accommodated state responsively to the natural accommodation mechanism of the eye, without the need for external power. The resting state of the lens implant is typically the fully-accommodated state, or, optionally, slightly beyond the fully-accommodated state, such that the lens implant is always pressing the lens capsule open even when the lens implant is fully accommodated, thereby keeping the zonules in tension.

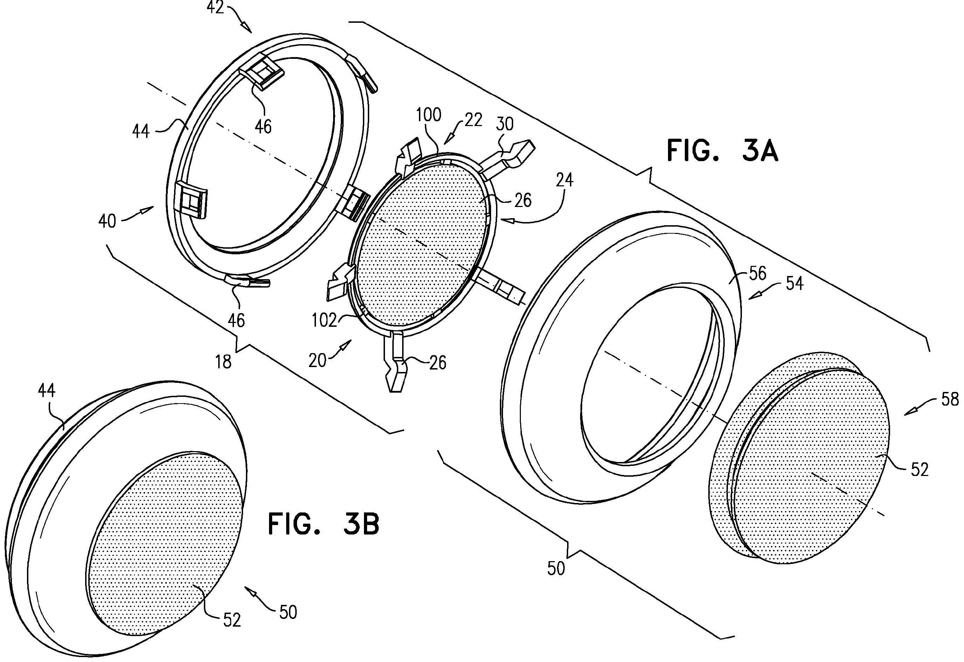

Reference is still made to Figs. 1A-C and 2A-C, and is additionally made to Figs. 3A-B, which are schematic illustration of components of lens implant 10 prior to assembly and after assembly, respectively, in accordance with an application of the present invention.

Lens implant 10 comprises (a) an anterior assembly 18 and (b) a posterior lens unit 50, which comprises a posterior lens 52. For some applications, anterior assembly 18 comprises:
- a first anterior component 20, which typically comprises exactly one first polymeric piece 22, which is shaped so as to define (i) an anterior floating lens unit 24, which comprises an anterior lens 26, and (ii) levers 30; and
- a second anterior component 40, which (a) typically comprises exactly one second polymeric piece 42, (b) typically is separate and distinct from first anterior component 20 prior to assembly with first anterior component 20, and (c) is shaped so as to define (i) an anterior rim complex 44, and (ii) anterior rim links 46, which are connected to anterior rim complex 44.

Typically, first and second anterior components 20 and 40 are assembled together during the manufacturing process. Because first and second anterior components 20 and 40 are separate and distinct from each other prior to assembly together, first and second anterior components 20 and 40 are separable from each other without tearing first anterior component 20 and without tearing second anterior component 40. For some applications, first and second polymeric pieces 22 and 24 are manufactured by injection molding. First and second polymeric pieces 22 and 24 may be more readily molded as separate pieces that are later assembled together during manufacture.

As appropriate, lens implant 10 may partially or wholly comprise silicone, or lens implant 10 may partially or wholly comprise acrylic. For some applications, a portion of lens implant 10 comprises silicone and another portion of lens implant 10 comprises acrylic (optionally, lens implant 10 consists essentially entirely of silicone and acrylic).

For some applications, posterior lens rim 56 comprises acrylic, which is typically flexible, any may be either hydrophobic or hydrophilic. Acrylic has shape memory, like silicone, but acrylic returns to its memorized shape more slowly than silicone, which provides more control during the implantation procedure than does silicone. Alternatively or additionally, first polymeric piece 22 comprises acrylic, and/or second polymeric piece 42 comprises acrylic.

It is noted that having lens implant 10 comprise acrylic (e.g., by consisting essentially entirely of acrylic) solves problems associated with use of silicone, such as "flash" (silicone leakage during injection molding). Even known flashless silicone molding techniques pose technical challenges when utilized with mold shapes that are complex. In addition, since suitable acrylic has a refractive index of up to about 1.52, while a suitable commercially-available silicone has a refractive index of 1.43, use of an acrylic lens as anterior lens 26 allows anterior lens 26 to be smaller than if anterior lens 26 were to comprise silicone; the same benefit may be achieved by posterior lens 52 if it comprises acrylic.

For some applications, (a) the material of first polymeric piece 22 has a hardness of between 20 and 70 Shore A, (b) the material of second polymeric piece 42 has a hardness of between 20 and 70 Shore A, (c) the material of first posterior component 54 has a hardness of between 20 and 70 Shore A, and (d) the material of a second posterior component 58 has a hardness of between 20 and 70 Shore A. Thus, all components of lens implant 10 are typically flexible.

For some applications, anterior assembly 18 and posterior lens unit 50 are distinct from each other and not permanently fixed to each other, and are shaped so as to be assemblable together *in situ* in capsular bag 12 of a human eye.

For some applications, lens implant 10 comprises between three and eight levers 30, such as three, four, five, or six levers 30, and a corresponding number of anterior rim links 46.

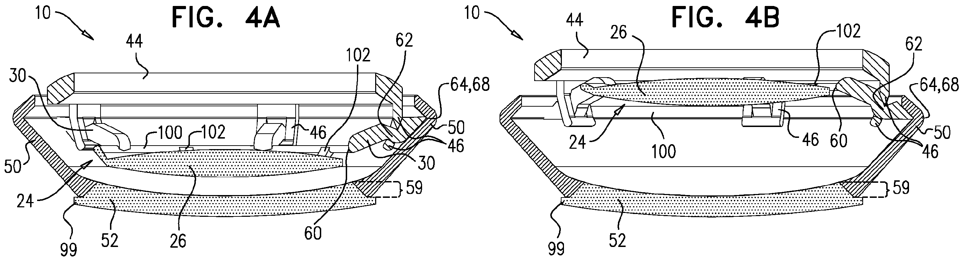

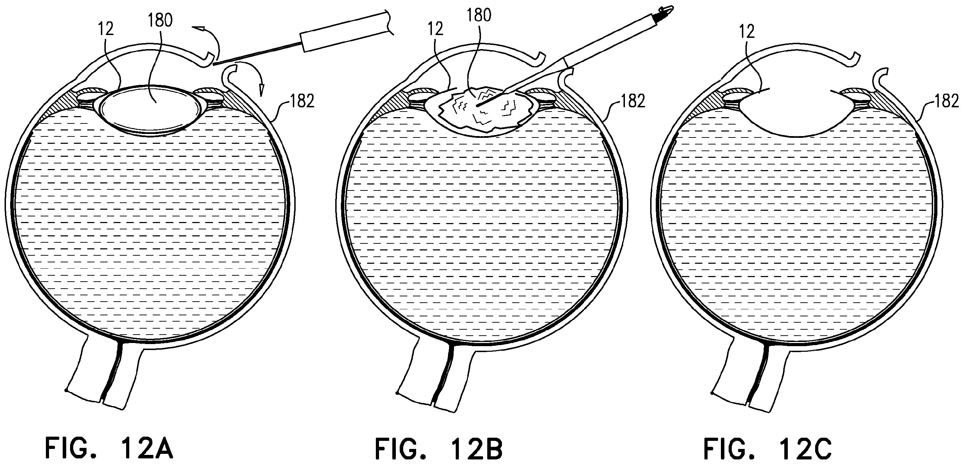

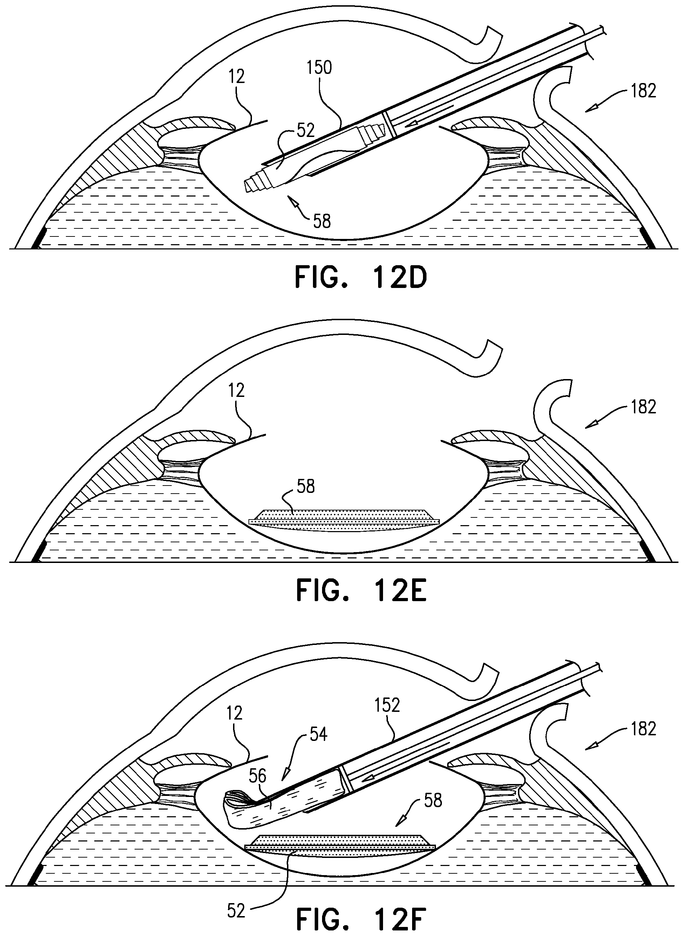

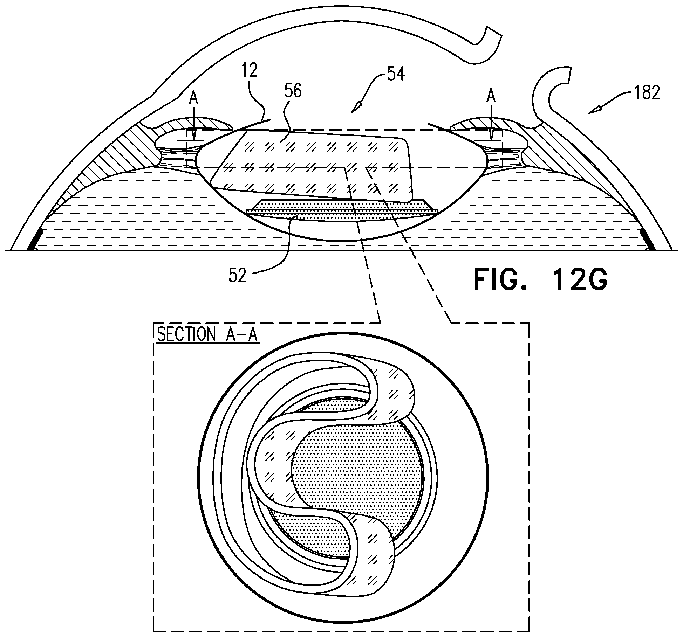

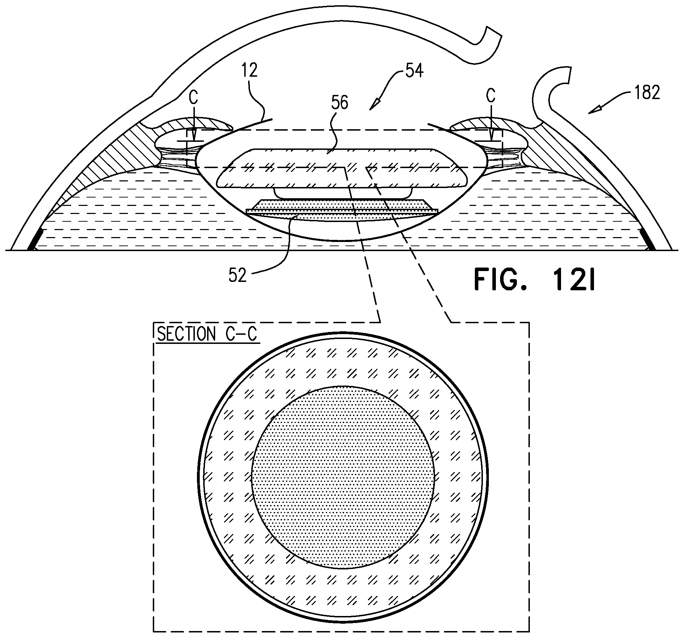

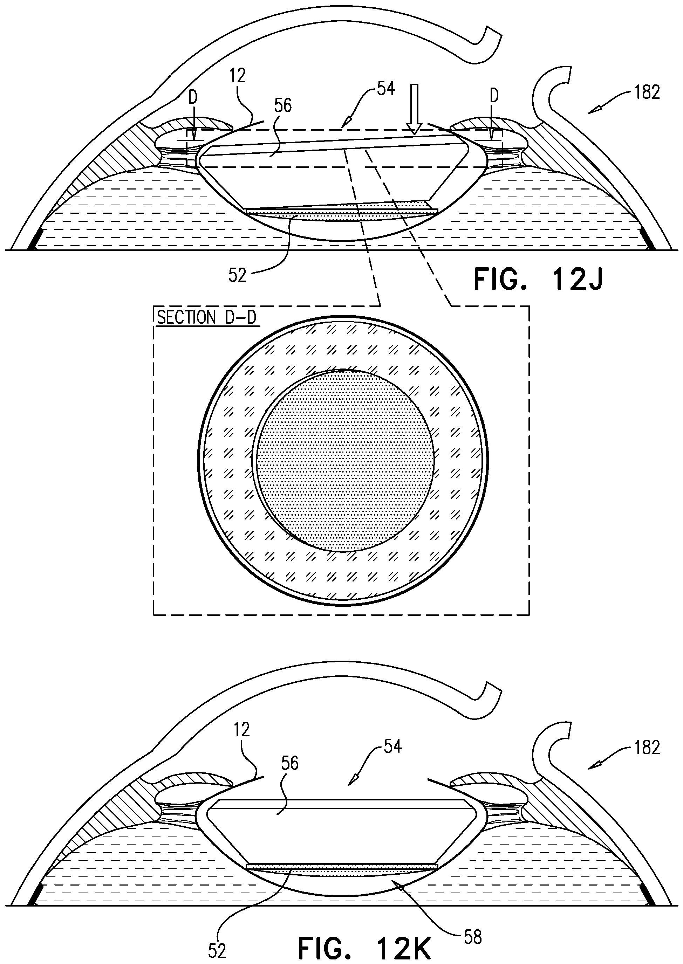

For some applications, posterior lens unit 50 comprises (a) a first posterior component 54, which comprises a posterior lens rim 56, and (b) a second posterior component 58, which (i) is distinct and separate from first posterior component 54, and (ii) comprises posterior lens 52. Because first and second posterior components 54 and 58 are separate and distinct from each other prior to assembly together, first and second posterior components 54 and 58 are separable from each other without tearing first posterior component 54 and without tearing second posterior component 58. As described hereinbelow with reference to Figs. 12F and 12G, posterior lens 52 and posterior lens rim 56 are shaped so as to be assemblable together *in situ* in capsular bag 12 of a human eye such that posterior lens rim 56 radially surrounds at least an axial portion 59 of posterior lens 52 (labeled in Fig. 4A). (As used in the present application, including in the claims, "axial" means a direction along a central optical axis of lens implant 10. As used in the present application, including in the claims, "radial" means in a direction toward or away from the central optical axis of lens implant 10.) (Although transparent, anterior lens 26 and posterior lens 52 are shaded in the figures for clarity of illustration; the lenses may comprise the same material as some or all the other components of the lens implant.)

Posterior lens unit 50 remains generally motionless with respect to the posterior portion of natural capsular bag 12 of the eye during accommodation of lens implant 10. Lens implant 10 is configured such that anterior floating lens unit 24 moves with respect to posterior lens unit 50 in response to the natural accommodation mechanism of the eye. The natural accommodation mechanism of the eye changes the shape of natural capsular bag 12, as shown in Figs. 2A-C. In the fully-unaccommodated state shown in Fig. 2A, the ciliary muscle is relaxed and the zonular fibers are therefore tensed, causing the capsular bag to assume a relatively narrow width (in an anterior-posterior direction) and relatively large diameter. Thus shaped, the capsular bag squeezes the lens implant in the anterior-posterior direction. In contrast, in the fully-accommodated state shown in Fig. 2C, the ciliary muscle contracts, thereby releasing the tension of the zonular fibers on the capsular bag, causing the capsular bag to assume a relatively large width and relative small diameter. This shape of the capsular bag allows the lens implant to expand in the anterior-posterior direction. (As used herein, the diameter of the capsular bag means the greatest diameter of the capsular bag when viewed from its posterior aspect.)

Anterior rim complex 44 is disposed such that anterior floating lens unit 24 is movable toward and away from anterior rim complex 44, in the anterior-posterior direction. As the width (in the anterior-posterior direction) of the capsular bag changes, anterior rim complex 44 moves with respect to posterior lens unit 50, thereby changing the distance therebetween.

As mentioned above, anterior floating lens unit 24 comprises anterior lens 26, and posterior lens unit 50 comprises posterior lens 52. Each of lens units 24 and 50 may comprise one or more additional optical elements, such as additional lenses (e.g., convex lenses, concave lenses, biconvex lenses, biconcave lenses, spherical lenses, aspheric lenses, and/or astigmatic lenses), fixed power optics, deformable optics, aberration free optics, doublets, triplets, filtered optics, or combinations of these lenses, as is known in the optical arts. For some applications, anterior lens 26 is the only optical element of anterior floating lens unit 24, and/or posterior lens 52 is the only optical element of posterior lens unit 50. For some applications, one or more of lens units 24 and 50 are attached to the implant during manufacture. Alternatively or additionally, one or more of the lens units may be attached by a healthcare worker either prior to or during the implantation procedure, such as to provide the lens unit most appropriate for the particular patient.

Reference is now made to Figs. 4A-B, which are schematic cross-sectional illustrations of lens implant 10 in the fully-unaccommodated state and the fully-accommodated state, respectively, in accordance with an application of the present invention. Levers 30 are arranged to move anterior floating lens unit 24 toward and away from anterior rim complex 44, in an anterior-posterior direction. Levers 30 are:
- in jointed connection with anterior floating lens unit 24 at respective first longitudinal sites 60 along levers 30,
- in jointed and interlocked connection with anterior rim links 46, respectively, at respective second longitudinal sites 62 along levers 30, and
- in jointed connection with posterior lens unit 50 at respective third longitudinal sites 64 along levers 30.

As used in the present application, including in the claims, a "lever" is a beam that is used to move an object at a first point by a force applied at a second point, and that pivots about a fulcrum at a third point. For each respective lever 30 of levers 30, the second longitudinal site 62 is longitudinally between first longitudinal site 60 and third longitudinal site 64 along the respective lever 30, such that third longitudinal site 64 serves as a fulcrum 66 for respective lever 30, and respective lever 30 is thus in pivotable contact with posterior lens unit 50 at respective third longitudinal sites 64. Thus, first longitudinal site 60, second longitudinal site 62, and third longitudinal site 64 correspond with the first, second, and third points, respectively, in the definition above. Typically, anterior rim links 46 pivot with reference to respective levers 30, such that levers 30 are in pivotable connection with anterior rim complex 44 via anterior rim links 46. Typically, during accommodation of lens implant 10 in the patient's eye, levers 30 and anterior rim links 46 do not bend or deform, but instead move with respect to each other. Typically, levers 30 are in interlocked connection with anterior rim links 46.

For some applications, third longitudinal sites 64 are at respective end-most sites 68 of respective levers 30. (The phrase "along" lever 30 is to be understood as including the ends of the lever; for example, third longitudinal site 64 may be at one end of the lever, as shown.) For some applications, each of levers 30 is in jointed connection with posterior lens unit 50 at an end-most site of the lever 30, when posterior lens unit 50 and first anterior component 20 are assembled together, typically *in situ.* Alternatively or additionally, for some applications, respective third longitudinal sites 64 along levers 30 directly contact posterior lens unit 50.

Force is applied to second longitudinal site 62 by anterior rim complex 44, and, as a result, first longitudinal site 60 (and anterior floating lens unit 24) moves more than an anterior-posterior distance that second longitudinal site 62 (and anterior rim complex 44) moves, typically between 1.5 and 4 times the anterior-posterior distance that second longitudinal site 62 (and anterior rim complex 44) moves. For some applications, a distance between second and third longitudinal sites 62 and 64 is between 0.8 and 1.6 mm, and a distance between first and third longitudinal sites 60 and 64 is between 1.2 and 2.4 mm, providing a gain of between 1.5 and 4. Typically, second longitudinal sites 62 are disposed radially inward from third longitudinal sites 64, respectively. Typically, first longitudinal sites 60 are disposed radially inward from second longitudinal sites 62 and third longitudinal sites 64, respectively.

Levers 30 are thus configured to magnify the relatively small change in the distance between anterior rim complex 44 and posterior lens unit 50, in order to move anterior floating lens unit 24 by a greater distance with respect to posterior lens unit 50. In other words, lens implant 10 is configured such that levers 30 move anterior floating lens unit 24 by a first anterior-posterior distance with respect to posterior lens unit 50 when anterior rim complex 44 moves a second anterior-posterior distance with respect to posterior lens unit 50, which first distance is greater than the second distance. Because of this distance magnification, the lens implant provides a high level of accommodation that mimics that of the natural eye. Typically, the first distance is at least 1.4 times the second distance, i.e., the lever provides a gain of at least 1.4. For example, the first distance may be at least 1.5 (e.g., at least 1.8, such as between 1.8 and 3) times the second distance.

The anterior and posterior movement of anterior floating lens unit 24 changes the distance between the anterior and posterior lens units, thereby adjusting the focal length of the lens implant. In the fully-accommodated state, which provides near vision, lens implant 10 is relatively wide (in the anterior-posterior direction), with a large separation between the anterior and posterior lens units, creating a large free space between the complexes. In the fully-unaccommodated state, which provides distance vision, the implant is relatively narrow, with a small separation between anterior and posterior complexes. Anterior floating lens unit 24 typically shifts at least 1 mm between the fully-unaccommodated and fully-accommodated states. Typical movement of the anterior lens relative to the posterior lens is between 0.5 and 2.0 mm, such as between 1 and 1.5 mm, as the lens implant transitions between the fully-unaccommodated and fully-accommodated states.

Anterior floating lens unit 24 moves within an interior space of lens implant 10, which is typically open to the natural fluid within the eye. The floating lens unit is configured to create minimum drag during movement, while maintaining the optical performance of the combined lens structure. For example, the floating lens unit may have a smooth shape, and/or may be coated with a hydrophobic coating such as silicone. Typically, the anterior and posterior lens units are configured to together create an optical structure having a total power that varies between +15D and +25D, as selected by the physician implanting the lens implant.

To minimize posterior capsular opacification, posterior lens 52 is typically provided with a clearly-defined corner 99 (e.g., having an angle of 80-150 degrees, e.g., 90-120 degrees), at the junction of the posterior and lateral surfaces of posterior lens 52.

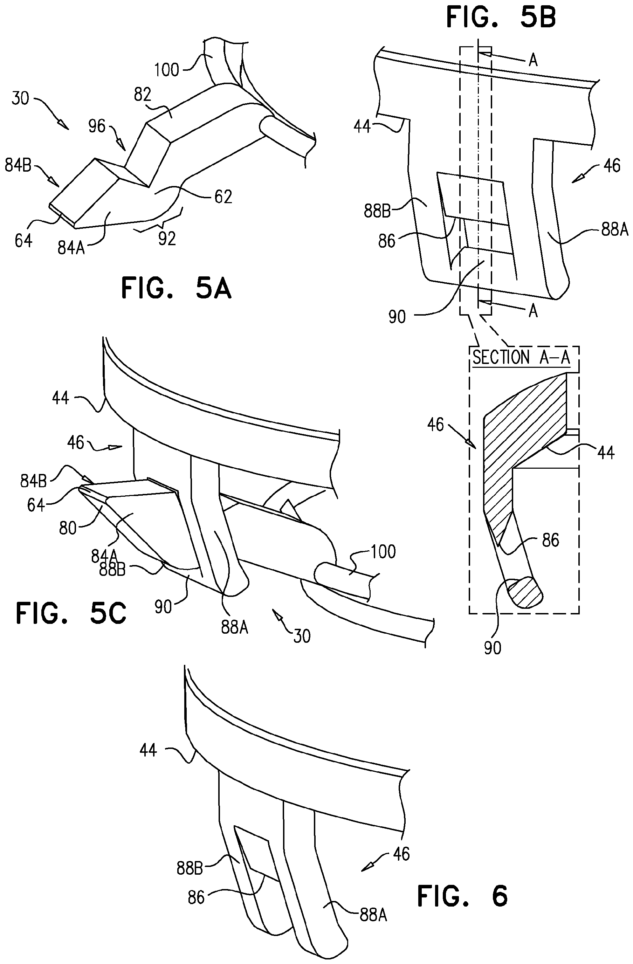

Reference is now made to Fig. 5A-C, which are schematic illustrations of one lever 30 and one anterior rim link 46, in accordance with an application of the present invention. Fig. 5A shows lever 30, Fig. 5B shows anterior rim link 46, and Fig. 5C shows anterior rim link 46 coupled to lever 30. For some applications, levers 30 are shaped so as to define respective posterior sides 80, respective anterior sides 82, and respective first and second lateral sides 84A and 84B (second lateral side 84B is not visible in Figs. 5A and 5C). Anterior rim links 46 are shaped so as to define:
- respective lever-contact surfaces 86, which are shaped and disposed so as to pivotably engage respective anterior sides 82 of levers 30 at respective second longitudinal sites 62 along levers 30, and
- respective first and second side arms 88A and 88B, which extend more posteriorly than respective lever-contact surfaces 86.
First side arms 88A are disposed at least partially alongside respective first lateral sides 84A of respective levers 30, and second side arms 88B are disposed at least partially alongside respective second lateral sides 84B of respective levers 30.

For some applications, such as shown in Figs. 5B-C, anterior rim links 46 are shaped so as to define respective posterior end pieces 90, which link respective first side arms 88A to respective second side arms 88B posteriorly beyond respective first and second lateral sides 84A and 84B. Typically, posterior end pieces 90 do not come in contact with respective posterior sides 80 of the respect levers at any point during a transition of accommodating intraocular lens implant 10 between the fully-accommodated state and the fully-unaccommodated state. Nevertheless, posterior end pieces 90 may still be provided, such as in order to hold anterior rim links 46 coupled to levers 30 during storage and implantation of first anterior component 20. For some applications, posterior sides 80 of levers 30 are convexly curved at least along 0.2 mm (e.g., at least along 0.3 mm) of respective levers 30 longitudinally surrounding respective second longitudinal sites 62. For some applications, respective convexly-curved portions 92 of posterior sides 80 of respective levers 30 are concentric with respective second longitudinal sites 62 of respective levers 30.

(As used in the present application, including in the claims, transitioning between the fully-accommodated and the fully-unaccommodated states is to be understood as meaning making a transition that begins at the fully-accommodated state and continues all the way to the fully-unaccommodated state, or vice versa.)

For some applications, levers 30 are shaped so as to define respective indentations 96 on respective anterior sides 82 at respective second longitudinal sites 62, and anterior rim links 46 are shaped and disposed so as to pivotably engage respective indentations 96.

Reference is now made to Fig. 6, which is a schematic illustration of another configuration of one anterior rim link 46, in accordance with an application of the present invention. In this configuration, anterior rim links 46 are not shaped so as to define respective posterior end pieces 90. (Nevertheless, levers 30 are still in interlocked connection with anterior rim links 46.)

Reference is made to Figs. 5A-B and 6. As described hereinabove, lens implant 10 may comprise acrylic, in whole or in part. A technical problem associated with using acrylic rather than silicone is that acrylic is less elastic than silicone, and therefore is liable to break if it undergoes local deformations that may be experienced during the transition between the fully-accommodated and the fully-unaccommodated states. In this regard, lens implant 10 (and particularly the interaction of lever 30 and anterior rim link 46 as shown in Figs. 5A-B and 6) addresses the technical challenge of creating lens implant 10 using acrylic, by providing lever 30 and anterior rim link 46 formed by molding as separate pieces that are later assembled together during manufacture. Thus, lever-contact surface 86 and anterior side 82 of lever 30 pivot with respect to each other to facilitate the desired transition between the fully-accommodated and the fully-unaccommodated states, substantially without bending of lever-contact surface 86 and/or anterior side 82 of lever 30.

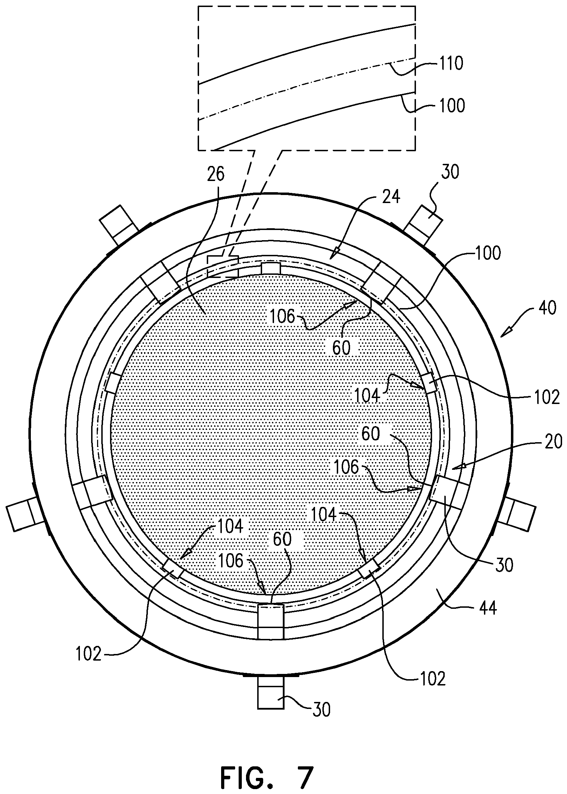

Reference is now made to Fig. 7, which is a schematic anterior view of first and second anterior components 20 and 40 of lens implant 10, in accordance with an application of the present invention. For some applications, anterior floating lens unit 24 further comprises:
- a ring 100 (e.g., an anterior lens ring 100), which has a diameter greater than that of anterior lens 26; and
- rim-connector elements 102, which are distributed around anterior lens ring 100 at respective connector sites 104, and connect anterior lens ring 100 to anterior lens 26 such that anterior lens ring 100 does not directly contact anterior lens 26.
Levers 30 are connected to respective lever-ring sites 106 around anterior lens ring 100, at respective first longitudinal sites 60 along levers 30. Typically, connector sites 104 are circumferentially offset from lever-ring sites 106. For example, each of lever-ring sites 106 may be circumferentially centered between two circumferentially-adjacent ones of connector sites 104, such as shown.

For some applications, accommodating intraocular lens implant 10 is configured such that ring 100 (e.g., anterior lens ring 100) deforms during a transition of accommodating intraocular lens implant 10 between the fully-accommodated state and the fully-unaccommodated state. For some applications, ring 100 has a circular longitudinal axis 110 around the anterior lens ring, and accommodating intraocular lens implant 10 is configured such that ring 100 twists (e.g., locally) around circular longitudinal axis 110 during the transition of accommodating intraocular lens implant 10 between the fully-accommodated state and the fully-unaccommodated state.

For some applications, accommodating intraocular lens implant 10 is arranged such that:
- elastic potential energy is stored in lens implant 10 as a result of deformation of lens implant 10 during a transition from the fully-accommodated state, such as shown in Figs. 1C, 2C, and 4B, to the fully-unaccommodated state, such as shown in Figs. 1A, 2A, and 3A (elastic potential energy is of course also stored during any decrease in accommodation of the lens implant), and
- at least 50% (e.g., at least 70%, such as at least 90%) of the elastic potential energy stored in lens implant 10 as the result of the deformation is stored in ring 100.

In other words, ring 100 functions as a spring that provides at least 50% (e.g., at least 70%, such as at least 90%) of the energy storage of lens implant 10 during the transition from the fully-accommodated state to the fully-unaccommodated state.

As a result of this springiness, the resting state of the lens implant is typically the fully-accommodated state, or, optionally, slightly beyond the fully-accommodated state, such that the lens implant is always pressing the lens capsule open even when the lens implant is fully accommodated, thereby keeping the zonules in tension.

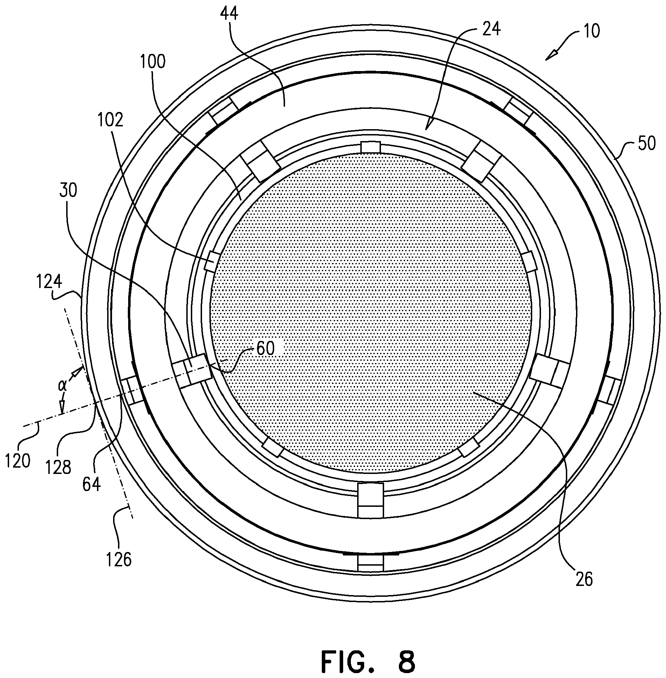

Reference is made to Fig. 8, which is a schematic anterior view of lens implant 10, in accordance with an application of the present invention. For some applications, for each respective lever 30 of levers 30, (a) a straight line 120 defined by first longitudinal site 60 of the respective lever 30 and third longitudinal site 64 of the respective lever 30, when projected onto a plane defined by a radially-outer perimeter 124 of accommodating intraocular lens implant 10, and (b) a straight line 126 that is in the plane and that is tangential to radially-outer perimeter 124 at a circumferential site 128 of radially-outer perimeter 124 circumferentially corresponding to third longitudinal site 64 of respective lever 30, form an angle α (alpha) of between 75 and 105 degrees, such as 85 to 95 degrees, e.g., 90 degrees (as shown).

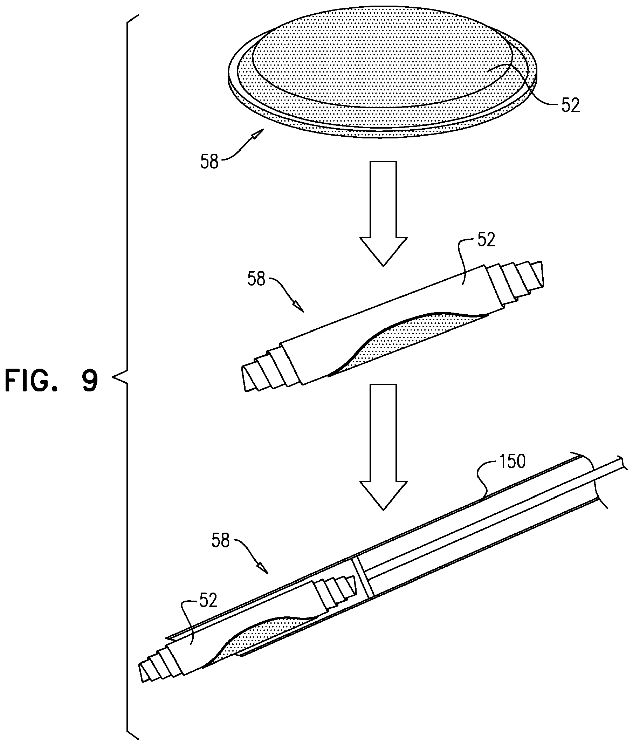

Reference is made to Fig. 9, which is a schematic illustration of the removable disposal of second posterior component 58 of lens implant 10 in a first introducer tube 150, in accordance with an application of the present invention. As shown in Fig. 9, second posterior component 58 (which comprises posterior lens 52) is removably disposed in first introducer tube 150 while rolled.

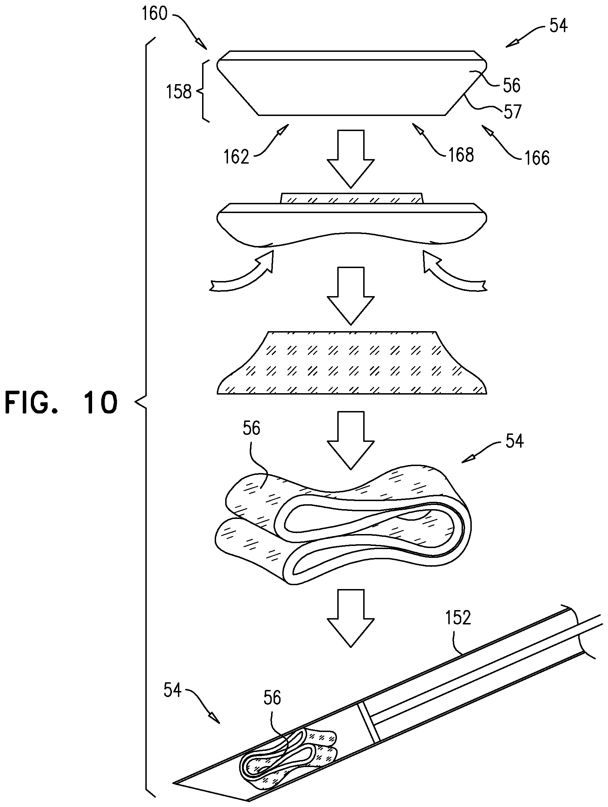

Reference is made to Fig. 10, which is a schematic illustration of the removable disposal of posterior lens rim 56 of first posterior component 54 of lens implant 10 in a second introducer tube 152, in accordance with an application of the present invention. For some applications, as shown in Fig. 10, posterior lens rim 56 of first posterior component 54 is removably disposed in second introducer tube 152 while folded or rolled. For some applications, posterior lens rim 56, when in an unconstrained state, includes a frustoconical portion 158 and defines larger and smaller openings 160 and 162. Posterior lens 52 is insertable into and coupleable with smaller opening 162. For some applications, posterior lens rim 56 is removably disposed in second introducer tube 152 while posterior lens rim 56 is inverted from its unconstrained state and folded or rolled. For some applications, posterior lens rim 56, when in the unconstrained state, is shaped so as to define a posterior surface 57 which is convex (configuration not shown), rather than a posterior surface 57 which is strictly frustoconical as shown.

For some applications, posterior lens rim 56 is removably disposed in second introducer tube 152 while folded in half (configuration not shown). For some applications, posterior lens rim 56, when in an unconstrained state, is shaped as a partial bowl 166 surrounding an opening 168 into which posterior lens 52 is insertable; posterior lens rim 56 is placed (and removably disposed) in second introducer tube 152 while bowl 166 is inverted from its unconstrained state and folded in half.

For some applications, as shown in Fig. 10, posterior lens rim 56 is placed (and removably disposed) in second introducer tube 152 while folded in quarters. For some applications, posterior lens rim 56, when in an unconstrained state, is shaped as partial bowl 166 surrounding opening 168 into which posterior lens 52 is insertable; posterior lens rim 56 is removably disposed in second introducer tube 152 while bowl 166 is inverted from its unconstrained state and folded in quarters.

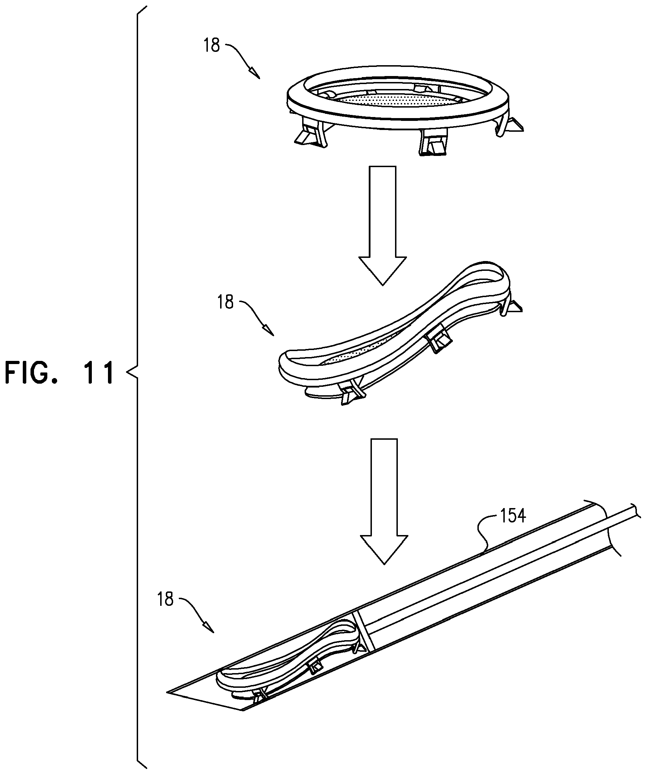

Reference is made to Fig. 11, which is a schematic illustration of the removable disposal of anterior assembly 18 of lens implant 10 in a third introducer tube 154, in accordance with an application of the present invention.

Reference is made to Figs. 12A-M, which are schematic illustrations of a method of implanting intraocular lens implant 10, in accordance with an application of the present invention. This three-step insertion procedure generally allows the use of a smaller incision than is necessary for a one-step insertion procedure of a single-piece implant. Typically, upon assembly, all of the rings and lenses of lens implant 10 are concentric.

As shown in Figs. 12A-C, a natural lens 180 is removed from a human eye 182, such as using conventional techniques known in the art. For example, as shown in Fig. 12B, an anterior capsulectomy may be made using continuous curvilinear capsulorhexis (CCC).

As shown in Figs. 12D-E, first introducer tube 150 is inserted into capsular bag 12 of eye 182, and second posterior component 58 (which comprises posterior lens 52) is released from the introducer tube in capsular bag 12 and positioned posteriorly in capsular bag 12.

As shown in Figs. 12F-K, second introducer tube 152 is inserted into capsular bag 12 of eye 182, and posterior lens rim 56 of first posterior component 54 is released from the introducer tube in capsular bag 12, is allowed to unfold or unroll in capsular bag 12, and is assembled *in situ* with second posterior component 58, such that posterior lens rim 56 radially surrounds at least axial portion 59 of posterior lens 52, as shown in Fig. 12K. For some applications, first and second posterior components 54 and 58 have matching beveled edges; second posterior component 58 is moved with respect to first posterior component 54 until the components align and become coupled together.

For some applications (configuration not shown), posterior lens rim 56 is introduced into capsular bag 12 folded or rolled, without being removably disposed in an introducer tube.

The scope of the present invention includes performing the steps of the method described with reference to Figs. 12F-K before performing the steps of the method described with reference to Figs. 12D-E.

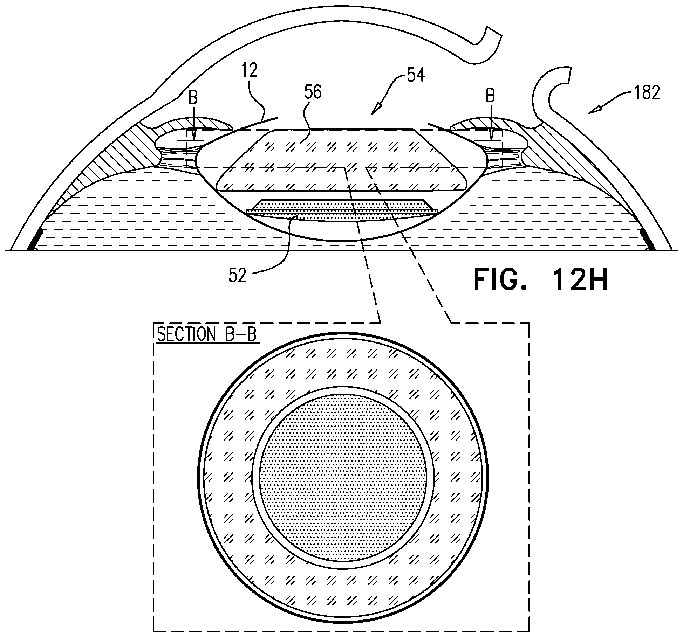

The inventors hypothesize that for many applications, the insertion of posterior lens rim 56 shown in Fig. 12F and the transition of the shape and position of posterior lens rim 56 from as shown in Fig. 12F to as shown in Fig. 12H are facilitated by posterior lens rim 56 having been inverted as shown in the sequence of steps in Fig. 10 prior to placement in second introducer tube 152. In particular, this inversion in some cases may reduce the likelihood of posterior lens rim 56 exiting the opening in capsular bag 12 after having been partially placed into capsular bag 12. The inversion shown in Figs. 12H-J may occur by itself, typically fairly quickly (because of the shape memory of the material), or may require some gentle prodding by the surgeon.

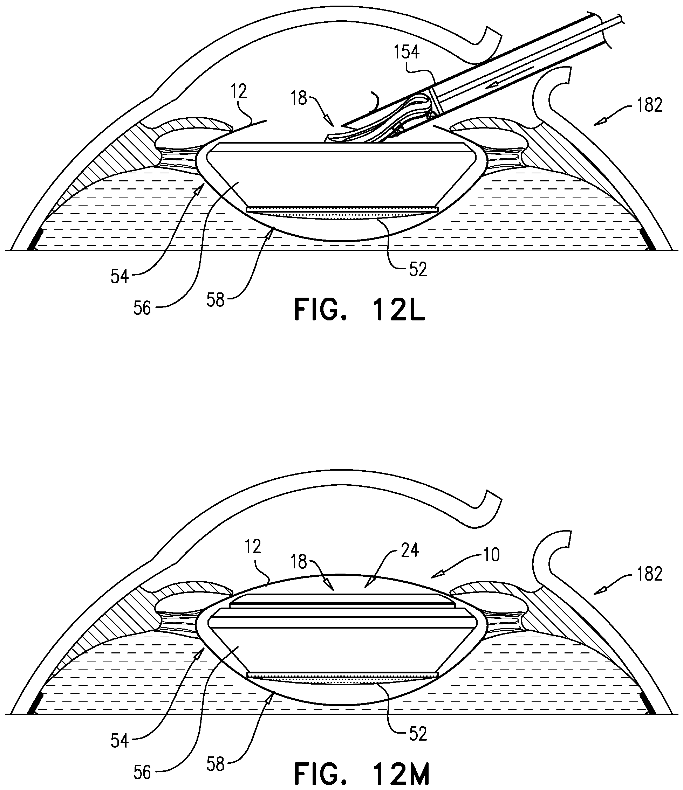

As shown in Figs. 12L-M, after first and second posterior components 54 and 58 have been released into capsular bag 12, third introducer tube 154 is inserted into capsular bag 12 of eye 182, and anterior assembly 18, including anterior lens 26 of anterior floating lens unit 24, is released from the introducer tube in capsular bag 12. Anterior floating lens unit 24 is coupled to second posterior component 58, as shown in Fig. 12M, thereby completing the implantation procedure.

For some applications, a single introducer tube is used to introduce all of the components of lens implant 10. Alternatively, for some applications, exactly two introducer tubes are used to introduce all of the components of lens implant 10, i.e., two of the components described above are introduced in a first introducer tube, and the third component is introduced in a second introducer tube.

For some applications, posterior lens 52 is inserted into capsular bag 12 after posterior lens rim 56 is released from introducer tube 150 in capsular bag 12. Alternatively, posterior lens 52 is inserted into capsular bag 12 before posterior lens rim 56 is released from introducer tube 150 in capsular bag 12.

Reference is now made to Figs. 1A-10I. For some applications, as shown in the figures, lens implant 10 does not comprise any haptics.

Although the two-part design of lens implant 10 has been described as being use for an accommodating IOL, the two-part design may also be used in non-accommodating and single lenses as well.

## Claims

1. Apparatus comprising an accommodating intraocular lens implant (10), which comprises:
an anterior floating lens unit (24), which comprises an anterior lens (26);
a posterior lens unit (50), which comprises a posterior lens (52);
an anterior rim complex (44);
levers (30), which are (a) (i) in jointed connection with the anterior floating lens unit (24) at respective first longitudinal sites (60) along the levers (30), (ii) in pivotable connection with the anterior rim complex (44) at respective second longitudinal sites (62) along the levers (30), and (iii) in pivotable contact with the posterior lens unit (50) at respective third longitudinal sites (64) along the levers (30), and (b) arranged to move the anterior floating lens unit (24) toward and away from the anterior rim complex (44), in an anterior-posterior direction; and
a ring (100), which has a diameter greater than that of the anterior lens (26), and which is connected to the levers (30),
wherein the accommodating intraocular lens implant (10) is configured such that the ring (100) deforms during a transition of the accommodating intraocular lens implant (10) between a fully-accommodated state and a fully-unaccommodated state,
**characterized in that** the accommodating intraocular lens implant (10) is arranged such that:
elastic potential energy is stored in the accommodating intraocular lens implant (10) as a result of deformation of the accommodating intraocular lens implant (10) during a transition from the fully-accommodated state to the fully-unaccommodated state, and
at least 50% of the elastic potential energy stored in the accommodating intraocular lens implant (10) as the result of the deformation is stored in the ring (100).

2. The apparatus according to claim 1,
wherein the ring (100) has a circular longitudinal axis (110) around the ring (100), and
wherein the accommodating intraocular lens implant (10) is configured such that the ring (100) twists around the circular longitudinal axis (110) during the transition of the accommodating intraocular lens implant (10) between the fully-accommodated state and the fully-unaccommodated state.

3. The apparatus according to claim 1, wherein the levers (30) and the posterior lens unit (50) are distinct from each other, and are shaped so as to be assemblable together *in situ* in a capsular bag of a human eye.

4. The apparatus according to claim 1, wherein, for each respective lever of the levers (30), the second longitudinal site (62) is longitudinally between the first and the third longitudinal sites (60, 64) along the respective lever (30), such that the third longitudinal site (64) serves as a fulcrum for the respective lever (30).

5. The apparatus according to claim 4, wherein the second longitudinal sites (62) are disposed radially inward from the third longitudinal sites (64), respectively, and the first longitudinal sites (60) are disposed radially inward from the second longitudinal sites (62) and the third longitudinal sites (64), respectively.

6. The apparatus according to claim 1, wherein the accommodating intraocular lens implant (10) is arranged such that at least 70% of the elastic potential energy stored in the lens implant (10) as the result of the deformation is stored in the ring (100).

7. The apparatus according to claim 6, wherein the accommodating intraocular lens implant (10) is arranged such that at least 90% of the elastic potential energy stored in the lens implant (10) as the result of the deformation is stored in the ring (100).

## Patentansprüche

1. Vorrichtung, die ein akkommodierendes Intraokularlinsenimplantat (10) umfasst, das Folgendes umfasst:
eine anteriore schwimmende Linseneinheit (24), die eine anteriore Linse (26) umfasst,
eine posteriore Linseneinheit (50), die eine posteriore Linse (52) umfasst,
einen anterioren Randkomplex (44),
Hebel (30), die (a) (i) sich in einer gelenkigen Verbindung mit der anterioren schwimmenden Linseneinheit (24) an jeweiligen ersten Längsseiten (60) entlang der Hebel (30), (ii) in einer schwenkbaren Verbindung mit dem anterioren Randkomplex (44) an jeweiligen zweiten Längsseiten (62) entlang der Hebel (30) und (iii) in einer schwenkbaren Berührung mit der posterioren Linseneinheit (50) an jeweiligen dritten Längsseiten (64) entlang der Hebel (30) befinden und (b) dafür angeordnet sind, die anteriore schwimmende Linseneinheit (24) hin zu und weg von dem anterioren Randkomplex (44), in einer Anterior-Posterior-Richtung, zu bewegen, und
einen Ring (100), der einen Durchmesser, größer als derjenige der anterioren Linse (26), aufweist und der mit den Hebeln (30) verbunden ist,
wobei das akkommodierende Intraokularlinsenimplantat (10) derart konfiguriert ist, dass sich der Ring (100) während eines Übergangs des akkommodierenden Intraokularlinsenimplantats (10) zwischen einem vollständig akkommodierten Zustand und einem vollständig nichtakkommodierten Zustand verformt,
**dadurch gekennzeichnet, dass** das akkommodierende Intraokularlinsenimplantat (10) derart angeordnet ist, dass:
elastische potenzielle Energie in dem akkommodierenden Intraokularlinsenimplantat (10) im Ergebnis einer Verformung des akkommodierenden Intraokularlinsenimplantats (10) während eines Übergangs von dem vollständig akkommodierten Zustand zu dem vollständig nichtakkommodierten Zustand gespeichert wird, und
mindestens 50 % der in dem akkommodierenden Intraokularlinsenimplantat (10) im Ergebnis der Verformung gespeicherten elastischen potenziellen Energie in dem Ring (100) gespeichert wird.

2. Vorrichtung nach Anspruch 1,
wobei der Ring (100) eine kreisförmige Längsachse (110) um den Ring (100) aufweist, und
wobei das akkommodierende Intraokularlinsenimplantat (10) derart konfiguriert ist, dass sich der Ring (100) während des Übergangs des akkommodierenden Intraokularlinsenimplantats (10) zwischen dem vollständig akkommodierten Zustand und dem vollständig nichtakkommodierten Zustand um die kreisförmige Längsachse (110) verdreht,

3. Vorrichtung nach Anspruch 1, wobei die Hebel (30) und die posteriore Linseneinheit (50) voneinander verschieden sind und so geformt sind, dass sie vor Ort in einem Kapselsack eines menschlichen Auges zusammenbaubar sind.

4. Vorrichtung nach Anspruch 1, wobei sich, für jeden jeweiligen Hebel der Hebel (30), die zweite Längsseite (62) längs zwischen der ersten und der dritten Längsseite (60, 64) entlang des jeweiligen Hebels (30) befindet, so dass die dritte Längsseite (64) als ein Drehpunkt für den jeweiligen Hebel (30) dient.

5. Vorrichtung nach Anspruch 4, wobei die zweiten Längsseiten (62) jeweils in Radialrichtung nach innen von den dritten Längsseiten (64) angeordnet sind und die zweiten Längsseiten (60) jeweils in Radialrichtung nach innen von den zweiten Längsseiten (62) und den dritten Längsseiten (64) angeordnet sind.

6. Vorrichtung nach Anspruch 1, wobei das akkommodierende Intraokularlinsenimplantat (10) derart angeordnet ist, dass mindestens 70 % der in dem Linsenimplantat (10) im Ergebnis der Verformung gespeicherten elastischen potenziellen Energie in dem Ring (100) gespeichert wird.

7. Vorrichtung nach Anspruch 6, wobei das akkommodierende Intraokularlinsenimplantat (10) derart angeordnet ist, dass mindestens 90 % der in dem Linsenimplantat (10) im Ergebnis der Verformung gespeicherten elastischen potenziellen Energie in dem Ring (100) gespeichert wird.

## Revendications

1. Appareil comprenant un implant de lentille intraoculaire adaptative (10), comprenant :
une unité de lentille flottante antérieure (24) comprenant une lentille antérieure (26) ;
une unité de lentille postérieure (50) comprenant une lentille postérieure (52) ;
un complexe de rebord antérieur (44) ;
des leviers (30), qui sont (a) (i) en connexion de liaison avec l'unité de lentille antérieure flottante (24) au niveau de premiers sites longitudinaux respectifs (60) le long des leviers (30), (ii) en connexion pivotante avec le complexe de rebord antérieur (44) au niveau de deuxièmes sites longitudinaux respectifs (62) le long des leviers (30), et (iii) en contact pivotant avec l'unité de lentille postérieure (50) au niveau de troisièmes sites longitudinaux respectifs (64) le long des leviers (30), et (b) configurés pour déplacer l'unité de lentille flottante antérieure (24) vers le complexe de rebord antérieur (44) et à l'écart de celui-ci, dans une direction antérieure-postérieure ; et
un anneau (100) ayant un diamètre supérieur à celui de la lentille antérieure (26) et connecté aux leviers (30) ;
dans lequel l'implant de lentille intraoculaire adaptative (10) est configuré de sorte que l'anneau (100) se déforme au cours d'une transition de l'implant de lentille intraoculaire adaptative (10) entre un état entièrement adapté et un état entièrement non adapté ;
**caractérisé en ce que** l'implant de lentille intraoculaire adaptative (10) est agencé de sorte que :
de l'énergie élastique potentielle est stockée dans l'implant de lentille intraoculaire adaptative (10) par suite d'une déformation de l'implant de lentille intraoculaire adaptative (10) au cours d'une transition de l'état entièrement adapté vers l'état entièrement non adapté ; et
au moins 50% de l'énergie élastique potentielle stockée dans l'implant de lentille intraoculaire adaptative (10) par suite de la déformation est stockée dans l'anneau (100).

2. Appareil selon la revendication 1,
dans lequel l'anneau (100) comporte un axe longitudinal circulaire (110) autour de l'anneau (100) ; et
dans lequel l'implant de lentille intraoculaire adaptative (10) est configuré de sorte que l'anneau (100) est tordu autour de l'axe longitudinal circulaire (110) au cours de la transition de l'implant de lentille intraoculaire adaptative (10) entre l'état entièrement adapté et l'état entièrement non adapté.

3. Appareil selon la revendication 1, dans lequel les leviers (30) et l'unité de lentille postérieure (50) son distincts les uns des autres et sont formés de sorte à pouvoir être assemblés in situ dans un sac capsulaire d'un œil humain.

4. Appareil selon la revendication 1, dans lequel, pour chaque levier respectif des leviers (30), le deuxième site longitudinal (62) se situe longitudinalement entre les premier et troisième sites longitudinaux (60, 64) le long du levier respectif (30), de sorte que le troisième site longitudinal (64) sert de point d'appui pour le levier respectif (30).

5. Appareil selon la revendication 4, dans lequel les deuxièmes sites longitudinaux (62) sont disposés respectivement radialement vers l'intérieur des troisièmes sites longitudinaux (64), les premiers sites longitudinaux (60) étant respectivement disposés radialement vers l'intérieur des deuxièmes sites longitudinaux (62) et des troisièmes sites longitudinaux (64).

6. Appareil selon la revendication 1, dans lequel l'implant de lentille intraoculaire adaptative (10) est agencé de sorte qu'au moins 70% de l'énergie élastique potentielle stockée dans l'implant de lentille (10) par suite de la déformation est stockée dans l'anneau (100) .

7. Appareil selon la revendication 6, dans lequel l'implant de lentille intraoculaire adaptative (10) est agencé de sorte qu'au moins 90% de l'énergie élastique potentielle stockée dans l'implant de lentille (10) par suite de la déformation est stockée dans l'anneau (100) .
